# EUROPÄISCHE PATENTANMELDUNG

(11) **EP 0 599 142 A2**
(43) Veröffentlichungstag der Anmeldung: **01.06.1994**
(21) Anmeldenummer: 93118266.1
(22) Anmeldetag: 11.11.1993
(51) Int. Cl.: G01N 33/531, G01N 33/94, A61K 31/34

(54) **Verfahren zur Herstellung von Immunkonjugaten**

(30) Priorität: 24.11.1992 DE 4239429
(71) Anmelder: MERCK PATENT GmbH, D-64293 Darmstadt (DE)
(72) Erfinder: Jalalian, Mohammad, Dr., D-64354 Reinheim 5 (DE); Heubner, Arnulf, Dr., D-55131 Mainz (DE); Reckmann, Bernd, Dr., D-64342 Seeheim (DE)

(57) **Zusammenfassung**

Die Erfindung betrifft ein neues Verfahren zur Herstellung von Immunkonjugaten aus in wäßriger Lösung schwer oder nicht löslichen Haptenen und Proteinen/Polypeptiden unter Verwendung von speziellen Lösungsmitteln, insbesondere Diethylenglykol-monoalkylethern.

## Beschreibung

Die Erfindung betrifft ein neues Verfahren zur Herstellung von Immunkonjugaten aus in wäßriger Lösung schwer oder nicht löslichen Haptenen und Proteinen/Polypeptiden gegebenenfalls unter Einbeziehung von Spacern bzw. Cross-Linkern.

Immunkonjugate spielen bei der Immundiagnostik und Immuntherapie eine immer bedeutendere Rolle. Dementsprechend wird auch auf funktionstüchtige und wirtschaftliche Verfahren zur Herstellung solcher Immunkonjugate Wert gelegt.

Nach den in der Standardliteratur beschriebenen Methoden wird ein Immunkonjugat durch Kopplung eines entsprechend modifizierten Haptens mit einem Protein oder Polypeptid direkt oder indirekt über geeignete Cross-Linker bzw. Spacer in einem wäßrigen, in der Regel gepufferten Medium hergestellt und anschließend nach Standardmethoden aufgereinigt.

Das Gelingen einer derartigen Kopplung ist dabei stark von der Löslichkeit des eingesetzten Haptens im wäßrigen (Puffer-)Medium abhängig. Viele Haptene, insbesondere Arzneimittel bzw. Arzneimittelderivate sind nun in Wasser bzw. wäßrigem Puffer nicht oder nur sehr schlecht löslich, wohl hingegen aber in den meisten gebräuchlichen organischen Lösungsmitteln. In organischen Lösungsmitteln denaturieren aber die meisten für diese Zwecke eingesetzten als Kopplungskomponenten dienenden Proteine/Polypeptide in zumeist irreversibler Weise, so daß keine biologisch wirksamen Konjugate erhalten werden können. Die Salzform, in die viele Haptene prinzipiell überführt werden können, ist zur Anwendung bei der Immunogensynthese in der Regel ungeeignet.

Es bestand somit die Aufgabe, ein Verfahren zur Herstellung von immunogen wirksamen Konjugaten zu entwickeln, welches es erlaubt, auch in wäßrigem Medium nicht oder nur schwerlösliche Haptene, insbesondere Arzneimittelderivate, einzusetzen.

Es wurde nun gefunden, daß bestimmte organische Lösungsmittel die in wärigem Medium schwerlöslichen Haptenkomponenten in befriedigender Menge zu lösen vermögen, ohne daß dabei die Proteinkomponente denaturiert wird.

Gegenstand der Erfindung ist somit ein Verfahren zur Herstellung von Immunkonjugaten, bestehend im wesentlichen aus einem in wäßriger Lösung schwerlöslichen Hapten (A) und einem Protein oder Polypeptid, gewünschtenfalls unter Einbeziehung eines Cross-Linkers oder Spacers (B), dadurch gekennzeichnet, daß die Kopplungsreaktion zwischen (A) und (B) im wesentlichen in einem oder einem Gemisch der nachfolgend aufgeführten Lösungsmittel durchgeführt wird:
- Diethylenglykol-monoalkylether, worin der Alkyl-Rest 1 bis 6 C-Atome aufweisen kann;
- Diethylenglykol-dialkylether, worin der Alkyl-Rest 1 bis 6 C-Atome aufweisen kann;
- 1,3-Dimethyl-2-imidazolidin-on bzw. 1,3-Dimethylimidazolidin-2-on (DMI);
- gamma-Butyrolacton;
- Glycerinformal oder
- 2,2-Dimethyl-4-hydroxymethy1-1,3-dioxolan

Es wurde auch gefunden, daß insbesondere solche Haptene geeignet sind, die einen chemisch reaktiven Rest aufweisen; insbesondere sind entsprechend modifizierte Amiodaronderivate geeignet.

Gegenstand der Erfindung ist somit ein entsprechendes Verfahren, daß dadurch gekennzeichnet ist, daß das Hapten eine Gruppe der Formel

-X-(CH₂)ₙ-Y,

worin
- X: O, S oder NR',
- Y: NHR', COR, SH, OH, CN oder Halogen,
- R: NH₂, Halogen oder OR',
- R': H oder Alkyl mit 1 bis 4 C-Atomen und
- n: eine ganze Zahl von 1 bis 10 bedeuten,
enthält.

Gegenstand der Erfindung ist insbesondere ein entsprechendes Verfahren, bei dem das Hapten ein Amiodaronderivat der Formel I ist, 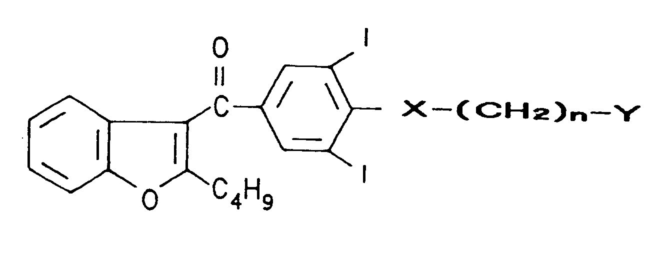
worin X, Y und n die angegebenen Bedeutungen haben.

Erfindungsgemäß bedeutet der Ausdruck "in wäßrigem Medium schwerlöslich", daß die Löslichkeit des entsprechenden Derivats in Wasser oder wäßrigem Puffer bei einem pH-Wert von etwa 6,5 bis 7,5 weniger als 200 mmol/l beträgt.

Die erfindungsgemäßen Lösungsmittel können einzeln aber auch im Gemisch miteinander eingesetzt werden. In diesen Lösungsmitteln können überraschend gut viele Haptenderivate, insbesondere die Verbindungen der Formel I, gelöst werden. Die genannten Lösungsmittel weisen überdies den erfindungswesentlichen Vorteil auf, daß sie sehr viele der für Kopplungsreaktionen gängigen Proteine/Polypeptide befriedigend zu lösen vermögen, ohne daß dabei eine wesentliche Denaturierung zu erkennen ist. Überdies haben die erfindungsgemäßen Lösungsmittel den Vorteil, daß sie mit Wasser mischbar sind, so daß auch die gängigen Cross-Linker bzw. Spacer, die gewünschtenfalls zwischen Hapten- und Proteinkomponente eingefügt werden sollen, bei der Kopplungsreaktion eingesetzt werden können. Besonders bevorzugt ist das Lösungsmittel Diethylenglykolmonoethylether (Carbitol®).

Die (in wäßrigem Medium schwer- bzw. unlöslichen) Haptenderivate werden zur Bereitstellung für die gewünschte Kopplungsreaktion in dem entsprechenden Lösungsmittel gelöst, wobei vorzugsweise Konzentrationen von 0,01 bis 3 mol/l eingestellt werden. Letztlich hängt die erreichbare Konzentration von der chemischen Natur des jeweiligen Haptens ab. Die Konzentration der bevorzugten Amiodaronderivate der Formel I in der jeweiligen Lösung beträgt vorzugsweise 0,01 bis 0,5 mol/l.

Als Haptenkomponente eignen sich alle (in wäßrigem Medium schwer- oder unlöslichen) Verbindungen, vorzugsweise Arzneimittelderivate oder Peptide, welche eine chemisch reaktive funktionelle Gruppe enthalten, die mit einem Amino-, SH-, OH- oder Carboxylrest der Protein-/Polypeptid-Komponente, zu reagieren im Stande sind. Inbesondere sind solche Derivate geeignet, die den Rest

-X-(CH₂)ₙ-Y,

worin
- X: O, S oder NR',
- Y: NHR', COR, SH, OH, CN oder Halogen,
- R: NH₂, Halogen oder OR',
- R': H oder Alkyl mit 1 bis 4 C-Atomen und
- n: eine ganze Zahl von 1 bis 10 bedeuten,
enthalten.

Alkyl bedeutet somit Methyl, Ethyl, Propyl, Isopropyl, n-Butyl, sec.-Butyl oder tert.-Butyl, vorzugsweise Methyl oder Ethyl. X ist vorzugsweise O oder NR' und Y ist vorzugsweise COR' bzw. NHR'. Halogen ist vorzugsweise Cl oder Br. n ist vorzugsweise 2 bis 6.

Derartige Reste sind in der Regel leicht nach Standardmethoden in das entsprechende Hapten-, bzw. Arzneimittelderivat einzuführen. Beispielsweise lassen sich phenolische OH-, SH- oder NHR-Gruppen enthaltende Haptenkomponenten mit Halogencarbonsäurederivaten zu den entsprechenden reaktiven, zur Konjugatbildung befähigten Verbindungen, beispielsweise den Amiodaronderivaten der Formel I, umsetzen.

Aber auch z. B. Reaktionen mit geeigneten aktivierten Doppelbindungen oder Aminogruppen sind gemäß an sich bekannter Methoden durchführbar.

Die für die Kopplungsreaktion vorgesehenen Proteine werden vorzugsweise in dem gleichen Lösungsmittel wie die Haptenkomponente gelöst, wobei die einzustellende Konzentration sich wiederum nach der chemischen Natur des Proteins richtet. Vorzugsweise werden Konzentrationen von 0,1-300 µmol/l, insbesondere 5-150 µmol/l, eingestellt.

Als Proteinkomponente eignen sich alle für diese Zwecke gängigen Proteine, beispielsweise Rinderserumalbumin (BSA), Keyhole limpet Haemocyanin (KLH), IgG, Ovalbumin, Lactalbumin oder Thyroglobulin.

Gewünschtenfalls können zwischen Hapten- und Protein/Polypeptid-Komponente für diese Zwecke allgemein bekannte Spacermoleküle bzw. Cross-Linker nach Standardmethoden eingefügt werden. Beispiele für solche Verbindungen sind N-Hydroxysuccinimid (NHS), N- (3-dimethylaminopropyl)-N'-ethylcarbodiimid-hydrochlorid (EDC), N,N'-Dicyclohexylcarbodiimid (DCC), 1-Cyclohexyl-3-(2-morpholinoethyl)carbodiimid (CMC) oder Verbindungen des Typs Z-(CH₂)ₙ-Y, worin Z XH oder Halogen bedeuten, und X und Y die angegebenen Bedeutungen haben. Vorzugsweise werden diese Verbindungen ebenfalls in dem gleichen Lösungsmittel wie die anderen beiden Komponenten gelöst oder aber zunächst in wenig Wasser gelöst und dann mit dem jeweiligen Lösungsmittel, welches die anderen Komponenten bereits in gelöster Form enthält, versetzt.

Erfindungsgemäß können die erfindungsgemäßen Lösungen aus Hapten- bzw. Protein- und gegebenenfalls Cross-Linker/Spacer-Komponente auch Wasser oder gängige andere organische Lösungsmittel, wie zum Beispiel DMSO, DMF, Methanol oder Ethanol, enthalten, welche ohne die erfindungsgemäßen Lösungsmittel in der Regel eine irreversibler Denaturierung des Proteins bewirken. Der Anteil dieser nicht erfindungsgemäßen Lösungsmittel richtet sich vorallem nach der Natur des Trägerproteins, beträgt aber in keinem Fall mehr als 50 % (v/v).

Der Anteil von Wasser darf nicht so hoch sein, daß die betreffende Hapten- oder auch Proteinkomponente auszufallen beginnt. Der Anteil von Wasser beträgt erfindungsgemäß 0-50 %, vorzugsweise 10-30 %.

Die erfindungsgemäße Immunogensynthese verläuft ansonsten unter Bedingungen, die aus der Standardliteratur bekannt sind (z.B. Colbert et al. (1986), Ann. Clinical Biochem. 23, 37).

In dem erfindungsgemäßen Verfahren werden als Haptenkomponente vorzugsweise Arzneimittelderivate, wie zum Beipiel die Verbindungen der Formel I eingesetzt. Eine Verbindung dieses Typs ist Amiodaron (2-Butyl-3-[3,5-diiodo-4-(β-diethylaminoethoxy)-benzoyl]benzofuran). Amiodaron findet in der Therapie als Antiarrhythmikum Verwendung. Weitere Beispiele für geeignete Arzneistoffe sind Phencyclidin, Tetrahydrocannabinol, Chinin oder Chinidin. Die nach dem erfindungsgemäßen Verfahren daraus hergestellten Immunkonjugate werden nach bekannten Methoden zur Immunisierung von zum Beispiel Mäusen verwendet. Die hierdurch gewonnenen Antikörper können so beispielsweise zum immunologischen Nachweis von Arzneimitteln, Arzneimittelderivaten oder Arzneimittelmetaboliten in humanen Körperflüssigkeiten verwendet werden.

### Beispiel 1

In 200 ml Acetonitril werden 32,8 g (60 mmol) 2-Butyl-3-[3,5-diiodo-4-hydroxy-benzoyl]-benzofuran und 13,8 g (100 mmol) wasserfreies Kaliumcarbonat vorgelegt. 17,6 g (90 mmol) Ethyl-4-brombutyrat werden bei Raumtemperatur innerhalb von 30 Minuten zugetropft und die Suspension anschließend 14 h unter Rückfluß erhitzt. Nach dem Abkühlen auf Raumtemperatur wird die Reaktionslösung mit 200 ml destilliertem Wasser versetzt und 3 mal mit 100 ml Methyl-tert.-butyl-Ether extrahiert. Die vereinten organischen Extrakte werden getrocknet und zur Trockene eingeengt. Der Rückstand, der aus 2-Butyl-3-[3,5-diiodo-4-(3-ethoxycarbonyl-propyloxy)-benzoyl]-benzofuran besteht, wird in 100 ml Isopropanol vorgelegt und eine Lösung von 39,6 g (600 mmol) 85 % igem Kaliumhydroxid in 400 ml destilliertem Wasser zugegeben. Die Suspension wird auf etwa 60 °C erwärmt bis sich eine klare Lösung bildet. Die auf Raumtemperatur abgekühlte Lösung wird mit 100 ml 1N Salzsäure versetzt, mit 32%iger Salzsäure unter Kühlung auf pH 3 eingestellt und mit 3 x 200 ml MTB-Ether extrahiert. Die vereinten organischen, getrockneten, einrotierten Extrakte werden in 50 ml Dichlormethan aufgenommen und für mehrere Stunden bei 0 °C belassen. Die ausgefallenen hellbraunen Kristalle werden mit weiteren 50 ml Dichlormethan verrührt. Nach Absaugen, Waschen und Trocknen erhält man 5,7 g (20 %) fast farblose Kristalle von 2-Butyl-3-[3,5-diiodo-4-(3-carboxy-propyloxy)-benzoyl]-benzofuran, welche gewünschtenfalls über einer Kieselgelsäule (MTB-Ether/ Methanol = 9 : 1) weiter aufgereinigt werden können.

### Beispiel 2

230 mg (0.36 mmol) des gemäß Beispiel 1 hergestellten Amiodaronderivats werden in 1,5 ml Carbitol® gelöst. Ebenso werden 50 mg (0,72 µmol) KLH in 25 ml Carbitol® zur Lösung gebracht. Die beiden Lösungen werden miteinander versetzt und 3 Stunden bei Raumtemperatur gerührt. Nach Beendigung der Reaktion wird der Ansatz gegen demineralisiertes Wasser bei 4 °C dialysiert. Nach der Dialyse wird die Lösung, welche das Konjugat aus Amiodaronderivat und KLH enthält, zur Immunisierung verwendet.

### Beispiel 3

Zu der Lösung des Amiodaronderivats gemäß Beispiel 2 werden 500 µl demineralisiertes Wasser, in dem 57 mg (0,3 mmol) EDC und 38 mg (0,33 mmol) NHS gelöst wurden, gegeben. Diese Lösung gibt man zu einer Lösung von 50 mg BSA in 25 ml Carbitol®. Anschließend wird gemäß Beispiel 2 verfahren. Man erhält ein Immunkonjugat aus dem Amiodaronderivat gemäß Beispiel 1, den genannten Cross-Linkern und BSA.

### Beispiel 4

Ein entsprechend nach Beispiel 1 hergestelltes Derivat aus Tetrahydrocannabinol und Br-(CH₂)₂-COOH wird in 2 ml Diglym gelöst (250 mg) und mit einer Proteinlösung aus 50 mg KLH in 30 ml Diglym versetzt. Anschließend wird gemäß Beispiel 2 verfahren. Man erhält ein Tetrahydrocannabinolderivat/KLH-Konjugat.

### Beispiel 5

Das nach Beispiel 1 hergestellte Amiodaronderivat (230 mg) wird in 3 ml Carbitol® gelöst. Zu dieser Lösung wird eine Lösung von 8-Ethoxycarbonyloctanol (Spacer) in 500 µl Methanol (1 : 4) gegeben. Diese Lösung wird mit einer Lösung von 50 mg KLH in 25 ml Carbitol® versetzt und wie beschrieben zur Reaktion gebracht. Man erhält ein entsprechendes Amiodaronderivat/Spacer/KLH- Konjugat.

### Beispiel 6

Nach Standardmethoden wird ein kopplungsfähiges Chininderivat hergestellt. Es wird gemäß Beispiel 2 ein Immunkonjugat mit BSA als Proteinkomponente hergestellt, wobei als Lösungsmittel ein Gemisch aus Carbitol®/DMI/Ethanol (40/40/20) verwendet wird.

### Beispiel 7

Aus dem Amiodaronderivat nach Beispiel 1 wird mit BSA und EDC (Cross-Linker) ein entsprechendes Immunkonjugat hergestellt, wobei als Lösungsmittel ein Gemisch aus Carbitol®/Glycerinformal/H₂O (50/15/35) verwendet wird.

## Patentansprüche

1. Verfahren zur Herstellung von Immunkonjugaten, bestehend im wesentlichen aus einem in wäßriger Lösung schwerlöslichen Hapten (A) und einem Protein oder Polypeptid, gewünschtenfalls unter Einbeziehung eines Cross-Linkers oder Spacers (B), dadurch gekennzeichnet, daß die Kopplungsreaktion zwischen (A) und (B) im wesentlichen in einem oder einem Gemisch der nachfolgend aufgeführten Lösungsmittel durchgeführt wird:
- Diethylenglykol-monoalkylether, worin der Alkyl-Rest 1 bis 6 C-Atome aufweisen kann;
- Diethylenglykol-dialkylether, worin der Alkyl-Rest 1 bis 6 C-Atome aufweisen kann;
- 1,3-Dimethyl-2-imidazolidin-on bzw. 1,3-Dimethylimidazolidin-2-on;
- gamma-Butyrolacton;
- Glycerinformal oder
- 2,2-Dimethyl-4-hydroxymethyl-1,3-dioxalan.

2. Verfahren nach Anspruch 1, dadurch gekennzeichnet, daß das Lösungsmittel Diethylenglykol-monoethylether ist.

3. Verfahren nach Anspruch 1 oder 2, dadurch gekennzeichnet, daß das Hapten eine Gruppe der Formel
-X-(CH₂)ₙ-Y,
worin
X O, S oder NR',
Y NHR', COR, SH, OH, CN oder Halogen,
R NH₂, Halogen oder OR',
R' H oder Alkyl mit 1 bis 4 C-Atomen, und
n eine ganze Zahl von 1 bis 10 bedeuten,
enthält.

4. Verfahren nach einem der Ansprüche 1-3, dadurch gekennzeichnet, daß das Hapten ein Arzneimittel ist.

5. Verfahren nach Anspruch 4, dadurch gekennzeichnet, daß ein Amiodaronderivat der Formel (I), 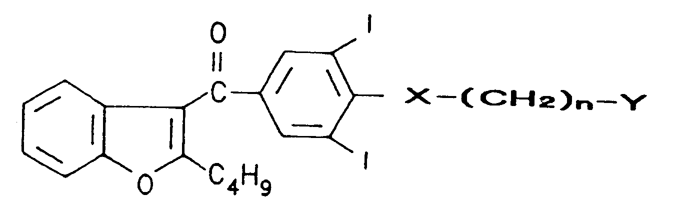 worin X, Y und n die angegebenen Bedeutungen haben, eingesetzt wird.
